# EUROPEAN PATENT SPECIFICATION

(11) **EP 0 721 776 B1**
(45) Date of publication and mention of the grant of the patent: **15.05.2002**
(21) Application number: 96300196.1
(22) Date of filing: 10.01.1996
(51) Int. Cl.: A61K 9/107, A61K 47/34

(54) **Electrostatic bonding type macromolecular micelle drug carrier and drug carried thereon**
Elektrostatisch bindender makromolekulärer Mizellen-Typ Wirkstoffträger mit daran gebundenem Wirkstoff
Véhicule macromoléculaire pour principe actif à liage électrostatique et principe actif porté là-dessus

(30) Priority: 10.01.1995 JP 221095
(43) Date of publication of application: 17.07.1996
(73) Proprietor: RESEARCH DEVELOPMENT CORPORATION OF JAPAN, Kawaguchi-shi, Saitama-ken 332 (JP)
(72) Inventor: Sakurai, Yasuhisa, Tokyo (JP); Okano, Teruo, Ichikawa-shi Chiba (JP); Kataoka, Kazunori, Chiba (JP); Yokoyama, Masayuki, Matsudo-shi Chiba (JP); Katayose, Satoshi, Kawasaki-shi Kanagawa (JP); Suwa, Satoru, Yamazaki Noda-shi Chiba (JP); Harada, Atsushi, 1495 Matsudo Matsudo-shi Chiba (JP)
(74) Representative: Calamita, Roberto

(56) References cited:
- EP-A- 0 303 516
- EP-A- 0 397 307
- EP-A- 0 440 100
- EP-A- 0 456 106
- EP-A- 0 552 802
- EP-A- 0 583 955
- CHEMICAL ABSTRACTS, vol. 124, no. 8, 1995 Columbus, Ohio, US; abstract no. 97318, K. KATAOKA: "PREPARATION OF NOVEL DRUG CARRIER BASED ON THE SELF-ASSOCIATION OF BLOCK COPOLYMER" XP002000095 & DRUG DELIVERY SYST., vol. 10, no. 5, 1995, pages 232-370, XP000566774 K. KATAOKA:
- MACROMOLECULES, vol. 28, no. 15, 17 July 1995, pages 5294-5299, XP000566344 A. HARADA AND K. KATAOKA: "FORMATION OF POLYION COMPLEX MICELLES IN AN AQUEOUS MILIEU FROM A PAIR OF OPPOSITELY-CHARGED BLOCK COPOLYMERS WITH POLY(ETHYLENE GLYCOL) SEGMENTS"

## Description

### FIELD OF THE INVENTION

The present invention relates to an electrostatic bonding type macromolecular micell drug carrier and drugs carried thereon. More particularly, the present invention relates to a novel macromolecular micell drug carrier of a chargeable drug such as protein and DNA, which is useful in areas such as a drug delivery system (DDS) which carries a drug to a permissive site in vivo and causes the drug to stably display the functions and effects thereof, drugs to be carried by such a carrier, and a method of carrying a drug on this carrier.

### PRIOR ART AND PROBLEMS

Macromolecular micell type drugs are attracting the general attention as a useful method for a drug delevery system (DDS), for example, and the present inventors have already proposed a macromolecular micell type drug which causes physical adsorption of a hydrophobic drug by a block copolyer comprising a hydrophilic segment and a hydrophobic segment.

The macromolecular micell type drug based on this physical adsorption is attracting the general attention because of a new structure and the possibility of using same in practice.

EP-583 955 discloses such bonding by hydrophobic interaction.

According to studies carried out by the present inventors, however, it is now clear that there still remain problems to be solved. More specifically, the macro-molecular micell drug based on this physical adsorption, although being very excellent as means to administer a hydrophobic drug, has a structure essentially characterized by physical adsorption of a hydrophobic drug by a block copolymer. There has therefore been a drawback that the method has been applicable only to drugs having a sufficient hydrophobicity.

Under such circumstances, there is a demand for achievement of novel technical means applicable in a wider range, which permits stable carrying of a drug irrespective of whether the drug is hydrophobic or hydrophilic.

### SUMMARY OF THE INVENTION

The present invention provides an electrostatic bonding type macromolecular micell drug carrier comprising a block copolymer having a non-chargeable segment and a chargeable segment, which solves the above-mentioned problems.

The present invention also provides embodiments of the above-mentioned carrier, in which the non-chargeable segment is polyethylene glycol; the chargeable segment is polyamino acid and the block copolymer is shown by any of the following formula (I) and (II); 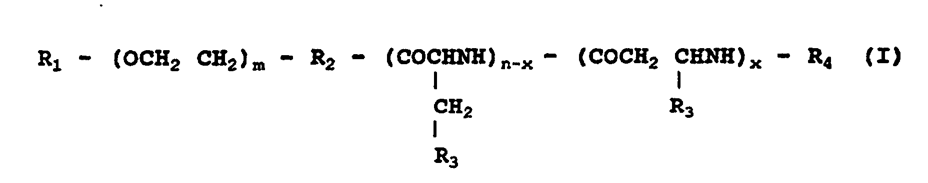 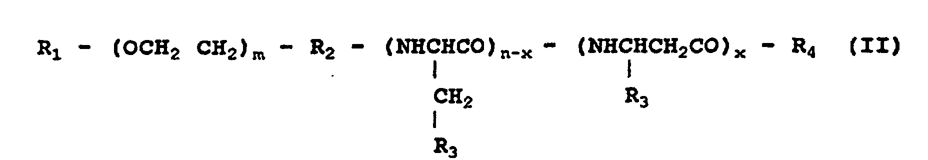 (where, R₁ is a hydrogen atom, a hydrocarbon group or a functional group or a functional group substituted hydrocarbon group; R₂ is NH, CO or R₆(CH₂)_{q}R₇, where R₆ indicates OCO, OCONH, NHCO, NHCOO, NHCONH, CONH or COO, R₇ indicates NH or CO, and q indictes an integerof 1 or more; R₃ is a carboxyl group, a carboxyl group substituted hydrocarbon group, an amino group substituted hydrocarbon group, a hydrazino group, substituted hydrocarbon group, (CH₂)ₚ-NHCNHNH₂ group, where p indicates an integerof 1 or more, a nitrogen-containing heterocyclic group or nitrogen-containing heterocyclic group substituted hydrocarbon group; R₄ is a hydrogen atom, a hydroxyl grooup or hydrocarbon group having any of CO, NH and 0 at thebonding terminal thereof; m is a number within a range of from 4 to 2,500; n is a number within a range of from 1 to 300; and x is a number within a range of from 0 to 300, provided that x < n).

In addition, the present invention provides an electrostatic bonding type macromolecular micell carrier drug in which a drug is carried by the carrier as described above, and a carrying method for the manufacture thereof.

### BRIEF DESCRIPTION OF THE DRAWINGS

Fig. 1 shows a spectral chart of 1H-NMR of PEG-P(Lys).

Fig. 2 shows a graph comparing measuring results of melting for cases with PEG-P(Lys)/DNA, free DNA and (Lys)/DNA.

### DETAILED DESCRIPTION OF THE INVENTION

The present invention as described above was developed as a result of studies carried out by the present inventors to overcome the problems in the conventional physical adsorption type macromolecular micell drug, and realizes a novel electrostatic bonding type macromolecular micell drug carrier essentially different from the physical adsorption type one, drugs carried by means thereof, and a method for carrying the drug.

In the electrostatic bonding type macromolecular micell carrier comprising a non-chargeable segment and a chargeable segment of the present invetnion as described above, various substances are applicable for the both segments within the scope of the present invention.

Applicable non-chargeable segments include, for example, polyalkylene glycol such as polyethylene glycol and plypropylene glycol, polyalkylene oxide, polysaccharide, polyacrylamide, poly-substituted acrylamide, polymethacrylamide, poly-substituted methacrylamide, polyvinylpyrrolidone, polyvinyl alcohol, polyacrylic acid ester, polymethacrylic acid ester, polyamino acid, and derivatives thereof.

Applicable chargeable segments include, for example, a polyamino acid having a chargeable side chain, or more specifically, polyaspartic acid, polyglutamic acid, plylysine, polyarginine, polyhistidine, or, polymalic acid, polyacrylic acid, polymethacrylic acid, polyethlene imine, polyvinylamine, polyacrylamine, polyvinyl imidazole, and drivatives thereof.

Substances applicable as a block copolymer of the present invention comprising these segments include;

Polyethylene glycol-polyaspartic acid block copolymer, polyethylene oxide-polyglutamic acid block copolymer, polyethylene glycol-polyarginine block copolymer, polyethylene glycol-polyhistidiine block copolymer, polyethylene glycolpolyhistidine block copolymer, polyethylene glycol-polymethacrylic acid block copolymer, polyethylene-polyvinylamine block copolymer, polyethylene glycol-plyarylamine block copolymer, polyethylene oxide-polyaspartic acid block copolymer, polyethylene oxide-polyglutamic acid block copolymer, polyethylene oxide-polylysine block copolymer, polyethylene oxide-polyarylic acid copolymer, polyethylene oxide-polyvinyl imidazole block copolymer, polyacrylamide-polyaspartic acid block copolymer, polyacrylamide-polyhistidine block copolymer, polymethacrylamide-polyarylic acid block copolymer, polymethacrylamide-polyvinylamine block copolymer, polyvinylpyrrolidone-polyaspartic acid block copolymer, polyvinylalcohol-polyarginine block copolymer, plyacrylic acid ester-polyhistidine block copolymer, polymethacrylic acid ester-polyvinylamine block copolymer, and polymethacrylic acid-polyvinylimidazole block copolymer.

A representative structure of these block copolymers is one known as AB-type block copolymer.

More specifically, the following paragraph describes an AB-type block copolymer comprising a non-chargeable segment obtained from a polyethylene glycol derivative and polyaspartic acid as the chargeable segment:

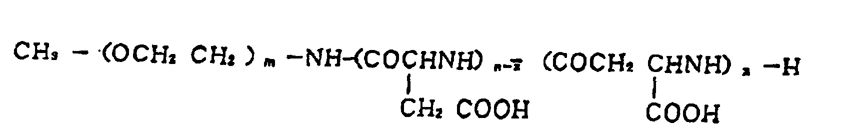

This is a polyethylene glycol-poly(α, β- aspartic acid) block copolymer comprising polyethylene glycol and poly(α,-β-aspartic acid), and is synthesized by copolymorizing β -benzyl-L-aspartate-N-carboxylic anhydride with poly-ethylene glycol which is a unilateral terminal aminogroup(molecular weight: 200 to 250,000) as the initiating agent. The molecular weight of the (β-benzyl, L-aspartate) portion of this polyethylene glycol (β -benzyl-L-aspartate) block copolymer is variable within a range of from about 205 to 62,000. Polyethylene glycol-poly(α,β-aspartic acid) block copolymer is available by eliminating benzyl through application of an alkali treatment of this copolymer.

Polyethylene glycol-polylysine block copolymer, shown by the following formula, having a cationic segment as the block copolymer: 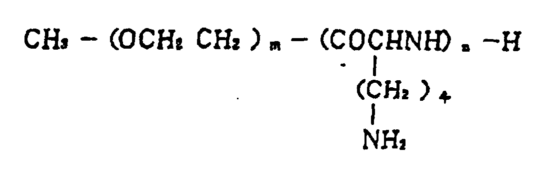 is synthesized through polymerization of ε-carbobenzoxy-L-lysine anhydride with unilateral terminal primary aminogroup polyethylene glycol (molecular weight: 200 to 250,000) as the initiating agent. Polyethylene glycol-polylysine block copolymer is available by subjecting the resultant polyethylene glycol-poly(ε-carbobenzoxy-L-lysine) block copolymer to a deprotecting reaction by the use of methane sulfonic acid.

In the present invention, while there is no particular limitation in the kind of drugs capable of being electrostatically carried in a macromolecular micell comprising a block copolymer as described above, applicable ones include macromolecular drugs such as peptide hormone, protein, DNA, RNA, and oligonucleotide and low molecular weight drugs having a chargeable functional group in molecules such as Adriamycin and Daranomycin.

When causing the macromolecular micell to carry any of these drugs, it is the basic practice to mix the block copolymer and the drug or a solution theref. Various operations including dialysis, stirring, dilution, concentration, ultrasonication, temperature control, pH control and addition of an organic solvent may appropriately be adapted.

When including lyoszyme, an antimicrobial enzyme, in the polyethylene glycol-poly(α, β-aspartic acid) block copolymer shown above, lysozyme can be carried by mixing an aqueous solution of the copolymer with an aqueous solution of lysozyme under appropriate conditions including mixing ratio, ionic strength and pH.

Furthermore, when causing the polyethylene glycol-polylysine block copolymer described above to carry DNA, it is possible to conducted DNA to be carried by mixing an aqueous solution of the copolymer with an aqueous DNA solution under conditions including appropriate mixing ratio, ionic strength and pH.

As described above, according to the electrostatic bonding type macromolecular micell drug carrier and the carried drug using same of the present invention, a stable macromolecular mice llstructure is available and chargeable substances such as protein and DNA can be efficiently incorporated into the internal nucleus therof. It is thus decomposed in vivo into the body in a stable state.

The present invention is now described further in detail by means of examples.

### Examle 1

Poly-L-lysine (degree of polymerization: 20,0.43 mg) was dissolved into distilled water (1.0ml), and a polyethylene glycol-polyaspartic acid block copolymer (PEG-P(Asp): molecular weight of PEG: 5,000, 23 aspartic acid residues per a chain of the block copolymer, 1.0 mg) was dissolved into distilled water (1.0 ml). Thereafter, these aqueous solutions were mixed. A weight average particle size of 41.3 nm and a number average particle size of 36.0 nm of the resultant mixture were measured by the method of dynamic light scattering. A zeta-potential of 0.643 and 0.569 mV for the entire surface of the mixture was measured by the method of trophoretic light scattering.

### Example 2

Polyaspartic acid (degree of polymerization: 20, 0.32 mg) was dissolved into distilled water (1.0 ml), and polyethylene glycol-poly-L-lysine block copolymer PEG-P(Lys); molecular (weight of PEG; 5,000, 20 L-lysine residues per chain of block copolymer, 1.0 mg) was dissolved into distilled water (1.0 ml). Thereafter, these aqueous solutions were mixed. A weight average particle size of 28.2 nm and a number average particle size of 42.8 nm of the resultant mixture were measured by the method of the dynamic light scattering.

### Example 3

Chicken albumen lysozyme (1.0 mg) was dissolved into distilled water (1.0 ml), and PEG-P(Asp) (3.0 mg) was dissolved into distilled water (3.0 ml). Thereafter, these solutions were mixed. A weight average particle size of 24.9 nm and a number average particle size of 23.1 nm of the resultant mixture were measured by the method of the dynamic light scattering.

### Example 4

Boving insulin (1.42 mg) was dissolved into a 0.0005N hydrochloric acid (1.5 ml), and PEG-P(Lys) having a particle size of 0,58 mg was dissolved into distilled water (1.0 ml). Thereafter, these solutions were mixed. A weight average particle size of 24.5 nm, and a number average particle size of 22.4 nm of the mixed solution were measured by the method of dynamic light scattering.

### Example 5

A polyethylene glycol-polylysine block copolymer was synthesized in accordance with the following formula:

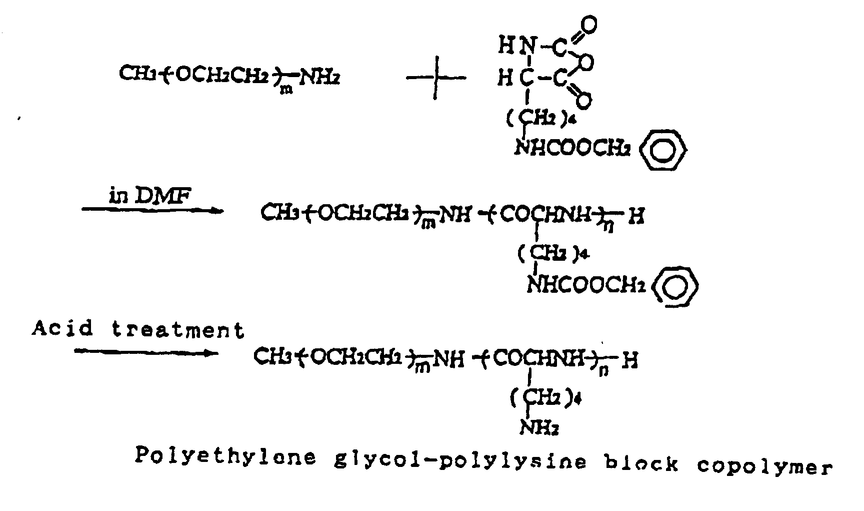

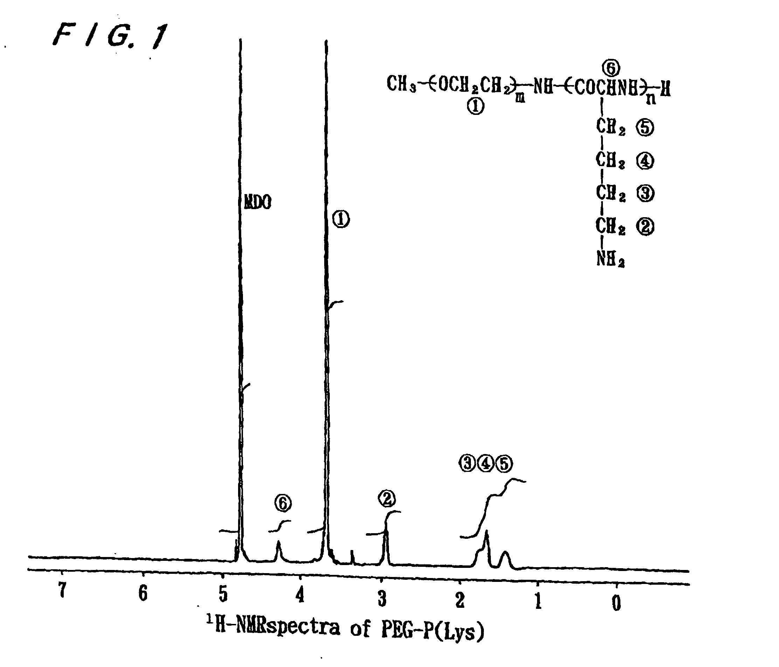

Fig. 1 shows ¹H-NMR spectra for a case with a PEG molecular weight of 4,300 and 20 L-lysine residues.

This PEG-P(Lys) block copolymer (PEG molecular weight; 4,300, average degree of polymerization of polylysine chain; 20) was dissolved into 1.0 ml of 0.1 M PBS (pH: 7.4) solution of Salmon Testes DNA in an amount of 50 ug/ml, and into 1.0 ml of 0.1 M PBS + 0.6 M NaCl + 2mM Na₂EDTA (pH: 7.4) so that the number of lysine residues of PEG-P(Lys) relative to DNA phosphte group became 0.25, 0.50, 1.0, 2.0, 4.0, 10 and 20 times as large, respecively. These solutions wore mixed and then held at the room temperature for three hours. No precipitation was observed in any of these samples. For a complex using polylysine homopolymer, on the other hand, precipitation took place in samples with ratios (=r) of lysine residues: DNA phosphate group of 1.0 and 2.0. Subsequently, a 20 µl fraction was taken from each sample and subjected to electrophoresis using 0.9% agarose gel. As a result, the amount of DNA migrating along with the increase in the amount of PEG-P(Lys) added to DNA decreased, and DNA migration was almost inhibited at an amount of addition (r = 1.0) of PEG-P(Lys) with wich the charge became equivalent to that of DNA. It was consequently confirmed that a quantitatively stable poly ion complex was formed by the PEG-P(Lys) block copolymer and DNA.

When using a plylysine homopolymer (molecular weight: 1,000 to 4,000) having a degree of polymerization almost equal to that Of the PEG-P(Lys) block copolymer, inhibition of DNA migration by addition of polylysine homopolymer was not observed and a stable complex was unavailable.

### Example 6

A PEG-P(Lys) block copolymer was dissolved into 1.0 ml of 1mM PBS (pH: 7.4) solution of Salmon Testes DNA in an aomount of 50 µg/ml, and into 1.0ml of 1mM PBS (pH: 7.4) so that the number of lysine residues of PEG-P(Lys) relative to DNA phosphate group became 0.10, 0.20, 0.50and 1.0 times as large, respectively. A complex was formed by mising these solutions. After holding the complex at 4°C for a night, the thermal melting curve of each sample was measured by adding methanol in an amount of 50 vol.X by the use of an ultraviolet absorban of 260 nm.

As a result, while the control DNA showed a first milting stage at about 45°C, the complex of DNA and PEG-P(Llys) showd two stage of melting at about 45°C and about 65°C. The increase in absorbance at about 45°C gradually decreased according as the amount of added PEG-P(Lys) was increased, whereas the increment of absorbance at about 65°C in that place. In the sample in which PEG-P(Lys) was added up to 1.0 times to DNA, the increase in absorbance at about 45°C diappears, and only the increase in absorbance at about 65°C was observed, suggesting that the structure of DNA was completely stabilized. This confirmed that DNA and PEG-P(Lys) stoichiometrically form a complex.

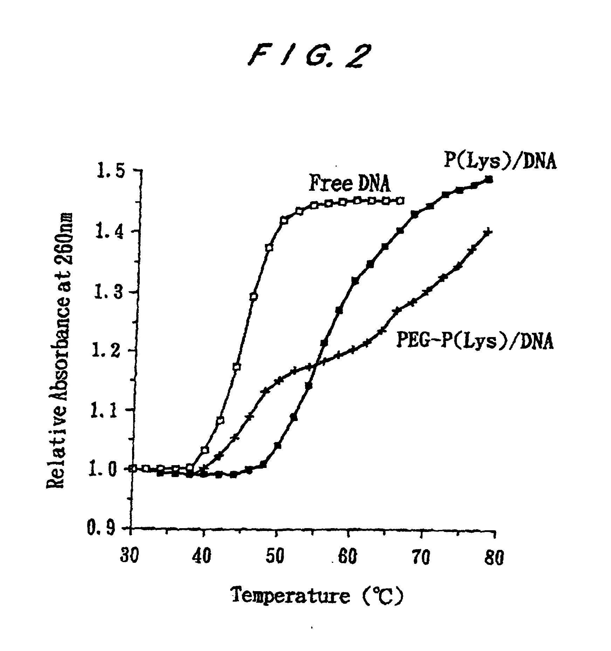

Fig. 2 shows a case where the number of lysine residues of PEG-P(Lys) is equal to 0.50 times relative to DNA phosphote group, and cases with free DNA and P(Lys)/DNA.

Remarkable differences are observed also in Fig. 2.

### Example 7

Poly-L-lysine (degree of polymerization: 20)(40 mg) was dissolved into 4 ml of the phosphate buffer solution, and polyethylene glycol-polyasparatic acid block copolymer(PEG-P(Asp);molecular weight of PEG: 5000, 20 aspharatic acid residues per a chain of the block copolymer, 2, 32mg) was dissolved into 2.32 ml of the phosphate buffer solution.

Thereafter, these aqueous solutions were mixed. A weight average particle size of 44.7 nm and a number average particle size of 41.3nm of the resultant mixture were measured by the method of dynamic light scattering.

### Example 8

Ploly-L-lysine (degree of polymerization:20) was dissolved into 4 ml of the phosphate buffer solution, and PEG-P(Asp)(molecular weight of PEG:5000, 80 asparatic acid residues per a chain of the block copolymer 4.5mg) was dissolved into 4.5 ml of the phosphate buffer solution. Therefore, these aqueous solution were mixed. A weight average particle size of 43.6 nm and a number average particle size of 41.8 nm of the resultant mixture are measured by the method of dynamic light scattering.

### Example 9

Polyethylene glycol-poly-L-lysine block copolymer(PEG-PLys:(molecular weight of PEG:500, 20 lysine residues per a chain of the block copolymer, 5mg) was dissolved into 1ml of the phosphate buffer solution, and polyethylene glycol-polyasparatic acid block copolymer(PEG-P(Asp): molecular weight of PEG: 5000, 20 asparatic acid residues per a chain of the block copolymer, 5mg, was dissolved into 1 ml of the phosphate buffer solution.

Thereafter, these aqueous solutions were mixed. A weight average particle size of 30.8 nm and a number average particle size of 28.8 nm of the resultant mixture were measured by the method of dynamic light scattering.

According to the present invention, as described above in detail, there are provided a carrier capable of stably carrying a drug under the effect of a macro molecular micell structure, and a drug carried by this carrier. Tt is possible to stably incorporate chargeable substances such as protein and DNA which tend to be easily decomposed in vivo.

## Claims

1. A macromolecular micelle carrier drug complex comprising a block copolymer macromolecular micelle drug carrier and a chargeable drug, wherein said block copolymer comprises a non-chargeable segment and a chargeable segment, **characterised in that** said chargeable drug has an opposite charge to said chargeable segment and is carried by electrostatic bonding to said chargeable segment.

2. A drug complex as claimed in claim 1 wherein said non-chargeable segment is polyethylene glycol.

3. A drug complex as claimed in claim 1 or claim 2 wherein said chargeable segment is polyamino acid.

4. A drug complex as claimed in claim 1 or claim 2 wherein said block copolymer comprises one of the following formulae (I) and (II): 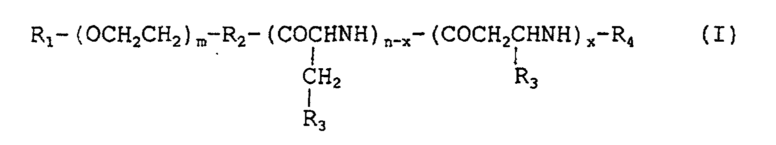 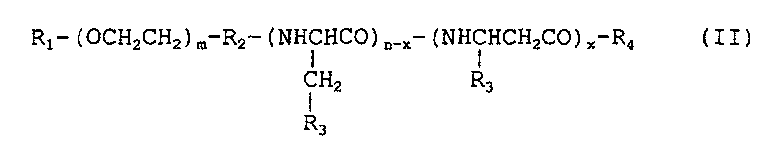 Wherein R₁ is a hydrogen atom, a hydrocarbon group or a functional group substituted hydrocarbon group; R₂ is NH, CO or R₆(CH₂)_{q}R₇, where R₆ indicates OCO, OCONH, NHCO, NHCOO, NHCONH, CONH or COO, R₇ indicates NH or CO, and q indicates an integer of 1 or more; R₃ is a carboxyl group, a carboxyl group substituted hydrocarbon group, an amino substituted hydrocarbon group, a hydrazino substituted hydrocarbon group, (CH₂)ₚ-NHCNHNH₂ group, where p indicates an integer of 1 or more, a nitrogen containing heterocyclic group or a nitrogen containing heterocyclic group substituted hydrocarbon group; R4 is a hydrogen atpm, a hydroxyl group or a hydrocarbon group having any of CO, NH and O at the bonding terminal thereof; m is a number within the range of from 4 to 2,500; n is a number within the range of from 1 to 300; and x is a number within the range of from 0 to 300, provided that x<n.

5. A drug complex as claimed in claim 4 wherein R₃ is a group chosen from the set of; -COOH, -CH₃COOH, -(CH₂)₃NH₂, -(CH₂)₂NHNH₂, or a heterocyclic group shown by the following formula:

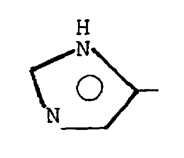

6. A drug complex as claimed in any of claims 1 to 5 wherein said chargeable drug is selected from the set of; peptide hormones, proteins, DNA, RNA, oligonucleotides and low molecular weight drugs with a chargeable functional group.

7. A method of forming a drug complex as specified in any of claims 1 to 6 comprising the steps of mixing said chargeable drug with said macromolecular micelle drug carrier having an opposite charge such that said drug is stably carried by said macromolecular carrier.

## Patentansprüche

1. Komplex aus Makromolekularmizellenträger und Wirkstoff bzw. Arzneimittel, der einen Blockcopolymer-Makromolekularmizellenwirkstoffträger und einen aufladbaren bzw. ionisierbaren Wirkstoff aufweist, wobei das Blockcopolymer ein nicht-aufladbares Segment und ein aufladbares Segment aufweist, **dadurch gekennzeichnet, daß** der aufladbare Wirkstoff eine entgegengesetzte Ladung zu dem aufladbaren Segment aufweist und durch elektrostatische Bindung von dem aufladbaren Segment getragen wird.

2. Wirkstoffkomplex nach Anspruch 1, wobei das nicht-aufladbare Segment Polyethylenglykol ist.

3. Wirkstoffkomplex nach Anspruch 1 oder Anspruch 2, wobei das aufladbare Segment Polyaminosäure ist.

4. Wirkstoffkomplex nach Anspruch 1 oder Anspruch 2, wobei das Blockcopolymer eine der folgenden Formeln (I) und (II) aufweist: 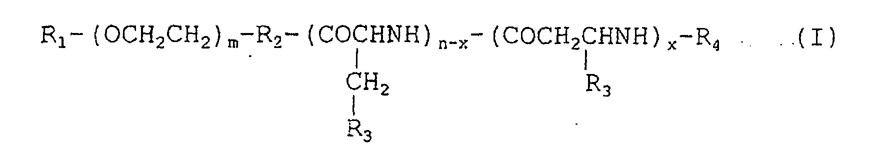 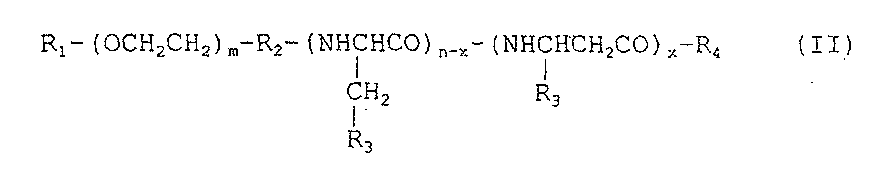 Wobei R1 ein Wasserstoffatom, eine Kohlenwasserstoffgruppe oder eine durch funktionelle Gruppen substituierte Kohlenwasserstoffgruppe ist; R₂ NH, CO oder R₆(CH₂)_{q}R₇ bedeutet, wobei R₆ OCO, OCONH, NHCO, NHCOO, NHCONH, CONH oder COO bedeutet, R₇ NH oder CO bedeutet und q eine ganze Zahl von 1 oder mehr bedeutet; R₃ eine Carboxylgruppe, eine durch Carboxylgruppen substituierte Kohlenwasserstoffgruppe, eine aminosubstituierte Kohlenwasserstoffgruppe, eine hydrazinosubstituierte Kohlenwasserstoffgruppe, eine Gruppe (CH₂)ₚ-NHCNHNH₂, wobei p eine ganze Zahl von 1 oder mehr darstellt, eine Stickstoff enthaltende heterozyklische Gruppe oder eine durch eine Stickstoff enthaltende heterozyklische Gruppe substituierte Kohlenwasserstoffgruppe ist; R4 ein Wasserstoffatom, eine Hydroxylgruppe oder eine Kohlenwasserstoffgruppe mit CO, NH oder O an deren Bindungsende bedeutet, m eine Zahl innerhalb des Bereichs von 4 bis 2.500 ist, n eine Zahl innerhalb des Bereichs von 1 bis 300 ist; und x eine Zahl innerhalb des Bereichs von 0 bis 300 ist, vorausgesetzt, daß x < n ist.

5. Wirkstoffkomplex nach Anspruch 4, wobei R₃ eine Gruppe ist, die aus der folgenden Reihe ausgewählt ist: -COOH, -CH₃COOH, -(CH₂)₃NH₂, -(CH₂)₂NHNH₂ oder eine heterozyklische Gruppe, die durch die folgende Formel dargestellt ist:

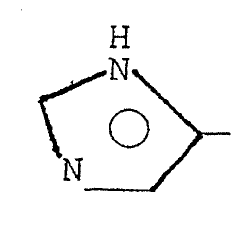

6. Wirkstoffkomplex nach einem der Ansprüche 1 bis 5, wobei der aufladbare Wirkstoff aus der folgenden Reihe ausgewählt ist: Peptidhormone, Proteine, DNA, RNA, Oligonukleotide und Wirkstoffe bzw. Arzneimittel mit niedrigem Molekulargewicht und einer aufladbaren bzw. ionisierbaren funktionellen Gruppe.

7. Verfahren zur Bildung eines Wirkstoffkomplexes nach einem der Ansprüche 1 bis 6, das die Schritte des Mischens des aufladbaren Wirkstoffs mit dem Makromolekularmizellenwirkstoffträger, der eine entgegengesetzte Ladung aufweist, umfaßt, so daß der Wirkstoff von dem makromolekularen Träger stabil getragen wird.

## Revendications

1. Complexe de médicament et de véhicule micellaire macromoléculaire, comprenant un véhicule micellaire macromoléculaire de type copolymère séquencé pour médicament et un médicament chargeable, ledit copolymère séquencé comprenant un segment non chargeable et un segment chargeable, **caractérisé en ce que** ledit médicament chargeable comporte une charge opposée à celle dudit segment chargeable, et est portée par ledit segment chargeable par l'intermédiaire d'une liaison électrostatique.

2. Complexe de médicament selon la revendication 1, dans lequel ledit segment non chargeable est de polyéthylène glycol.

3. Complexe de médicament selon la revendication 1 ou 2, dans lequel le segment chargeable est un polyaminoacide.

4. Complexe de médicament selon la revendication 1 ou 2, dans lequel ledit copolymère séquencé correspond à l'une des formules (I) et (II) suivantes : 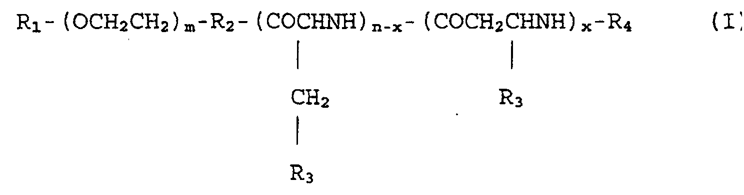 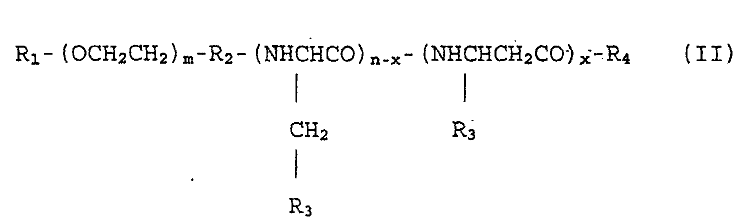 dans lesquelles R₁ représente un atome d'hydrogène, un groupe hydrocarboné ou un groupe hydrocarboné substitué par un groupe fonctionnel ; R₂ représente NH, CO ou R₆(CH₂)_{q}R₇, R₆ représentant OCO, OCONH, NHCO, NHCOO, NHCONH, CONH ou COO, R₇ représentant NH ou CO, et q représente un entier de 1 ou plus ; R₃ représente un groupe carboxy, un groupe hydrocarboné à substituant carboxy, un groupe hydrocarboné à substituant amino, un groupe hydrocarboné à substituant hydrazino, un groupe (CH₂)ₚ-NHCNHNH₂ dans lequel p représente un entier de 1 ou plus, un groupe hétérocyclique azoté ou un groupe hydrocarboné substitué par un groupe hétérocyclique azoté ; R4 représente un atome d'hydrogène, un groupe hydroxy ou un groupe hydrocarboné comportant un groupe CO, NH ou O à son extrémité de liaison ; m représente un nombre dans la plage de 4 à 2 500 ; n représente un nombre dans la plage de 1 à 300 ; et x représente un nombre dans la plage de 0 à 300, à condition que x < n.

5. Complexe de médicament selon la revendication 4, dans lequel R₃ représente un groupe choisi parmi -COOH, -CH₃COOH, -(CH₂)₃NH₂, -(CH₂)₂NHNH₂, ou un groupe hétérocyclique correspondant à la formule suivante :

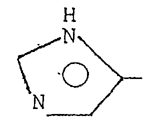

6. Complexe de médicament selon l'une quelconque des revendications 1 à 5, dans lequel ledit médicament chargeable est choisi parmi les hormones peptidiques, les protéines, l'ADN, l'ARN, les oligonucléotides et les médicaments de faible poids moléculaire à groupe fonctionnel chargeable.

7. Procédé de formation d'un complexe de médicament tel que spécifié dans l'une quelconque des revendications 1 à 6, comprenant les étapes consistant à mélanger ledit médicament chargeable avec ledit véhicule macromoléculaire micellaire pour médicament comportant une charge opposée, de sorte que ledit médicament est porté de manière stable par ledit véhicule macromoléculaire.
